# EUROPÄISCHE PATENTANMELDUNG

(11) **EP 1 647 244 A1**
(43) Veröffentlichungstag der Anmeldung: **19.04.2006**
(21) Anmeldenummer: 04024653.0
(22) Anmeldetag: 15.10.2004
(51) Int. Cl.: A61F 2/46

(54) **Instrumentensystem zum Einsetzen von Bandscheibenimplantaten**

(71) Anmelder: Zimmer GmbH, 8404 Winterthur (CH)
(72) Erfinder: Filippi, Michael, 8200 Schaffhausen (CH); Casutt, Guido, 8544 Sulz-Rickenbach (CH); Reinmuth, Jochen, 8406 Wintherthur (CH); Heller, Mathias, 8352 Räterschen (CH)
(74) Vertreter: Manitz, Finsterwald & Partner GbR

(57) **Zusammenfassung**

Die Erfindung betrifft ein Instrumentensystem zum Einsetzen von zwei Implantatplatten (11) und einen Implantatkern (13) umfassenden Bandscheibenimplantaten, mit wenigstens einer zwei vorzugsweise plattenförmige Probeelemente (17) umfassenden Probeeinheit (15) zum Bestimmen eines für den jeweiligen Patienten passenden Implantats, zumindest einer Bearbeitungseinheit (19) zum Vorbereiten der Wirbelkörperflächen des Patienten, die zwei vorzugsweise gabelförmige Abschnitte (21) aufweisende Arbeitselemente (23) umfasst, und wenigstens einer mit den Implantatplatten koppelbaren Halteeinheit (25) zum Einsetzen des Implantats, wobei die Probeeinheit, die Bearbeitungseinheit und die Halteeinheit zum Auseinanderdrücken der Probeelemente, der Arbeitselemente bzw. der Implantatplatten jeweils mit einem insbesondere zangenartigen Distraktionsinstrument koppelbar sind.

## Beschreibung

Die Erfindung betrifft ein Instrumentensystem zum Einsetzen von zwei Implantatplatten und einem Implantatkern umfassenden Bandscheibenimplantaten.

Bei derartigen Operationen kommt der Auswahl des richtigen, für den jeweiligen Patienten optimalen Bandscheibenimplantats eine entscheidende Bedeutung zu. Die Implantatauswahl erfolgt zu einem wesentlichen Teil präoperativ, z.B. mit Hilfe von Röntgenschablonen. Es lassen sich jedoch nicht immer alle Parameter des einzusetzenden Implantats präoperativ bestimmen. Insbesondere die optimale Höhe des Implantats lässt sich am geeignetsten intraoperativ ermitteln. Hierzu sind Distraktionsinstrumente erforderlich, mit denen die beiden benachbarten Wirbelkörper, zwischen die das Bandscheibenimplantat einzusetzen ist, aufgespreizt werden können. Ein Instrumentensystem zum Einsetzen von Bandscheibenimplantaten sollte dem Operateur also die Möglichkeit bieten, zur Auswahl des passenden Implantats eine Aufspreizung der betreffenden Wirbelkörper vorzunehmen. Gleichzeitig muss das für die Operation insgesamt erforderliche Instrumentarium überschaubar und leicht zu handhaben sein. Dies ist vor allem deshalb von Bedeutung, da das ausgewählte Implantat nicht sofort eingesetzt werden kann, sondern zuvor eine Vorbereitung des betreffenden Bandscheibenfachs erforderlich ist. Darüber hinaus sollte das Instrumentarium so wenig Platz wie möglich benötigen, um den zur Verfügung stehenden Arbeitsraum nicht unnötig zu verkleinern.

Aufgabe der Erfindung ist es, ein Instrumentensystem der eingangs genannten Art zu schaffen, das den vorstehend genannten Anforderungen gerecht wird, d.h. das bei zuverlässiger Funktionsweise überschaubar, möglichst einfach aufgebaut und für den Operateur möglichst einfach handhabbar ist, und das den Operateur bei jedem der einzelnen Operationsschritte so wenig wie möglich behindert.

Die Lösung dieser Aufgabe erfolgt erfindungsgemäß durch die Merkmale des Anspruchs 1 und insbesondere dadurch, dass das Instrumentensystem wenigstens eine zwei vorzugsweise plattenförmige Probeabschnitte umfassende Probeeinheit zum Bestimmen eines für den jeweiligen Patienten passenden Implantats, zumindest eine Bearbeitungseinheit zum Vorbereiten der Wirbelkörperflächen des Patienten, die zwei vorzugsweise gabelförmige Abschnitte aufweisende Arbeitselemente umfasst, und wenigstens eine mit den Implantatplatten koppelbare Halteeinheit zum Einsetzen des Implantats umfasst, wobei die Probeeinheit, die Bearbeitungseinheit und die Halteeinheit zum Auseinanderdrücken der Probeabschnitte, der Arbeitselemente bzw. der Implantatplatten jeweils mit einem insbesondere zangenartigen Distraktionsinstrument koppelbar sind.

Das erfindungsgemäße Instrumentarium ermöglicht die Durchführung aller erforderlichen Operationsschritte, nämlich eine abschließende, intraoperative Implantatauswahl, die anschließende Bearbeitung der Wirbelkörper sowie das Einsetzen des ausgewählten Implantats in das vorbereitete Bandscheibenfach. In jeder dieser Operationsphasen kann die dabei verwendete Instrumenteneinheit, d.h. die Probeeinheit, die Bearbeitungseinheit bzw. die Halteeinheit, mittels eines Distraktionsinstruments auseinandergedrückt werden. In der Probe- oder Auswahlphase besteht also die Möglichkeit, die abschließende Bestimmung des passenden Implantats bei einer dem natürlichen, gesunden Wirbelsäulenverlauf entsprechenden Aufspreizung der entsprechenden Wirbelkörper durchzuführen. Durch die Möglichkeit, die Arbeitselemente der Arbeitseinheit mittels eines Distraktionsinstruments auseinanderzudrücken, kann die Vorbereitung der Wirbelkörperflächen unter denjenigen Bedingungen erfolgen, unter denen zuvor die abschließende intraoperative Auswahl des Implantats durchgeführt wurde. Auch beim Einsetzen des Implantats werden die Wirbelkörper aufgespreizt, indem die mit der Halteeinheit gekoppelten Implantatplatten, die zuvor in das vorbereitete Bandscheibenfach eingeführt wurden, mittels eines Distraktionsinstruments auseinandergedrückt werden, so dass der Implantatkern problemlos zwischen die Implantatplatten eingebracht werden kann. Erfindungsgemäß basieren die Probeeinheit, die Bearbeitungseinheit und die Halteeinheit somit auf dem gleichen Arbeitsprinzip, nämlich dem Auseinanderdrücken der in der jeweiligen Arbeitsphase zum Einsatz kommenden Bestandteile des Instrumentensystems bzw. des Implantats. Aufgrund dieses gemeinsamen Distraktionsprinzips können die Einheiten derart ausgebildet werden, dass der Operateur lediglich ein einziges Distraktionsinstrument benötigt, das gegebenenfalls mittels entsprechender Adapterelemente an die Einheiten gekoppelt werden kann.

Vorzugsweise sind die Probeeinheit, die Bearbeitungseinheit und die Halteeinheit jeweils an ihrer während der Operation dem Operationsfeld abgewandten Rückseite mit Kopplungsabschnitten für das Distraktionsinstrument versehen. Der Platzbedarf in Richtungen senkrecht zur anterior-posterior-Richtung lässt sich hierdurch minimal halten, da der Operateu während der Operation mit dem Instrumentensystem bzw. dem Distraktionsinstrument gewissermaßen "im Schatten" des Implantats bleiben kann.

In einer besonders bevorzugten praktischen Weiterbildung der Erfindung weist die Probeeinheit eine einstellbare Tiefenlehre zur Festlegung einer Referenzposition für das Implantat in anterior-posterior-Richtung auf.

Hierdurch kann dem Umstand, dass die Auswahl eines für den jeweiligen Patienten optimal passenden Implantats auch von der Tiefe, d.h. der Position in anterior-posterior-Richtung, in welcher das Implantat einzusetzen ist, abhängig ist, Rechnung getragen und dafür gesorgt werden, dass möglichst großflächige Implantatplatten zum Einsatz kommen. Bei vergleichsweise schmalen Wirbelkörpern kann so versucht werden, die vorhandene Tiefe der Wirbelkörper so weit wie möglich auszunutzen und die Implantatplatten möglichst tief einzubringen, so dass das Drehzentrum möglichst weit nach posterior gelegen ist. Dagegen kann es beispielsweise bei vergleichsweise breiten Wirbelkörpern mit relativ geringer Tiefe angezeigt sein, zur Abdeckung einer möglichst großen Fläche Implantatplatten mit möglichst großen Abmessungen in anterior-posterior-Richtung zu verwenden, die nur in eine vergleichsweise geringe Tiefe eingebracht werden und deren nach anterior weisendes Ende folglich vergleichsweise nahe an der anterioren Kante des betreffenden Wirbelkörpers gelegen ist. Die im jeweiligen Fall optimale Lage des Implantats in anterior-posterior-Richtung kann erfindungsgemäß mit Hilfe der an der Probeeinheit vorgesehenen einstellbaren Tiefenlehre festgelegt werden. Die hierdurch erhaltene anterior-posterior-Einstellung, die im Folgenden auch kurz als AP-Einstellung bezeichnet wird, kann dann als Referenz für die weiteren Operationsschritte dienen.

Des Weiteren wird erfindungsgemäß vorgeschlagen, dass die Bearbeitungseinheit und/oder die Halteeinheit ebenfalls eine einstellbare Tiefenlehre aufweisen, die entsprechend der Referenzeinstellung der Probeeinheit einstellbar ist. Die Bearbeitung der Wirbelkörperflächen sowie das Einsetzen der Implantatplatten kann so mit Hilfe der auf die anfangs ermittelte Referenzposition eingestellten Tiefenlehren in einer auf das ausgewählte Implantat abgestimmten Weise erfolgen. Insbesondere ist sichergestellt, dass die Bearbeitungseinheit und die Halteeinheit jeweils die richtige AP-Position einnehmen. Insbesondere lässt sich durch die Tiefenlehre sicherstellen, dass sich die jeweils eingestellte AP-Position während der Bearbeitung der Wirbelkörperflächen bzw. beim Einsetzen der Implantatplatten nicht von selbst verändern kann.

Alle Tiefenlehren sind bevorzugt zumindest im Wesentlichen baugleich ausgeführt. Hierdurch wird das Instrumentensystem weiter vereinfacht.

Vorzugsweise ist die Tiefenlehre zumindest bereichsweise nach Art einer in anterior-posterior-Richtung relativ zur Probeeinheit verstellbaren Einstellschraube ausgebildet. Hierdurch kann eine beispielsweise mittels eines Schraubendrehers aufgebrachte Drehbewegung um eine parallel zur anterior-posterior-Richtung verlaufende Achse in eine in anterior-posterior-Richtung verlaufende Translationsbewegung der Tiefenlehre bzw. eines Abschnitts der Tiefenlehre relativ zur Probeeinheit umgesetzt werden.

In einer bevorzugten praktischen Ausgestaltung ist die Tiefenlehre über eine Stift/Nut-Führung mit der Probeeinheit gekoppelt. Die Verstellbewegung kann auf diese Weise durch eine Zwangsführung des Stiftes in der Nut erzielt werden.

Vorzugsweise weist die Tiefenlehre einen insbesondere mittels eines Schraubendrehers um eine im Wesentlichen parallel zur anterior-posterior-Richtung verlaufende Achse drehbaren Verstellabschnitt auf, an dem eine zumindest bereichsweise wendel- oder schraubenförmig um die Drehachse herum verlaufende Verstellnut für einen an der Probeeinheit angeordneten Führungsstift ausgebildet ist.

Der Verlauf der Verstellnut kann grundsätzlich beliebig ausgeführt sein. So kann die Verstellnut beispielsweise als Wendel mit einer konstanten Steigung ausgebildet sein. Alternativ ist es aber auch möglich, dass die Verstellnut eine Mehrzahl von aufeinander folgenden Nutabschnitten mit unterschiedlichen Steigungen umfasst.

Ferner wird vorgeschlagen, dass die Verstellnut mehrere voneinander beabstandete, vorgegebenen Tiefenpositionen entsprechende Rastvertiefungen für den Führungsstift aufweist. Hierdurch kann die Tiefenlehre eine Mehrzahl von diskreten Tiefen- oder Referenzkonfigurationen einnehmen, die stabil sind und nicht versehentlich gelöst werden können.

Gemäß einer weiteren besonders bevorzugten praktischen Ausgestaltung der Erfindung umfasst die Bearbeitungseinheit zusätzlich zu den Arbeitselementen wenigstens eine Führungshülse für ein Bearbeitungsinstrument, insbesondere einen Fräser oder Bohrer, wobei die Führungshülse zur Festlegung einer Bearbeitungsposition mit der Bearbeitungseinheit koppelbar ist. Die Kopplung erfolgt vorzugsweise derart, dass die Führungshülse in Richtungen senkrecht zur anterior-posterior-Richtung bezüglich der Bearbeitungseinheit festgehalten ist.

Die Bearbeitung der Wirbelkörperflächen in einer definierten, durch die Arbeitselemente vorgebbaren Höhenlage ist hierdurch gewährleistet.

Vorzugsweise ist die Führungshülse sowohl zur kaudalen als auch zur kranialen Bearbeitung ausgebildet und hierzu zunächst mit dem einen und anschließend mit dem anderen Arbeitselement koppelbar. Aufgrund dieser universellen Verwendbarkeit der Führungshülse ist das Vorsehen zweier unterschiedlicher Führungshülsen für die kaudale und die kraniale Bearbeitung der Wirbelkörper nicht erforderlich.

Ferner kann erfindungsgemäß vorgesehen sein, dass die Bearbeitungseinheit bereichsweise einen tunnelartigen Aufnahmeraum begrenzt, in den die Führungshülse zumindest teilweise in anterior-posterior-Richtung einschiebbar ist.

Gemäß einem weiteren bevorzugten Ausführungsbeispiel umfasst die Bearbeitungseinheit ein separates Verriegelungselement, mit dem ein mittels des Distraktionsinstrumentes hergestellter Abstand zwischen den Arbeitselementen fixierbar ist. Hierdurch übernimmt das Verriegelungselement die Aufrechterhaltung des distrahierten Zustandes, so dass das Distraktionsinstrument unter Beibehaltung des eingestellten Abstandes zwischen den Arbeitselementen entfernt und die Bearbeitung der Wirbelkörper durchgeführt werden kann, ohne dass der Operateur durch das Distraktionsinstrument in irgendeiner Weise behindert wird.

Vorzugsweise weist das Verriegelungselement wenigstens einen in anterior-posterior-Richtung zwischen die Arbeitselemente schiebbaren Verriegelungsabschnitt mit einem gestuften oder treppenartig verlaufenden Höhenprofil auf. Die Höheneinstellung an der Bearbeitungseinheit, d.h. das Aufspreizen der Wirbelkörper mittels der Arbeitselemente, kann hierdurch in diskreten Schritten insofern erfolgen, als während des Auseinanderdrückens der Arbeitselemente das Verriegelungselement nachgeschoben wird, sobald der Abstand zwischen den Arbeitselementen ausreichend groß ist, um die nächste Stufe des Höhenprofils verriegelnd zwischen die Arbeitselemente zu bringen.

Vorzugsweise ist der Verriegelungsabschnitt gabelförmig ausgebildet und umfasst zwei identische Höhenprofile aufweisende Verriegelungsarme, die jeweils zwischen einander zugewandte Längskantenabschnitte der vorzugsweise in diesem Bereich halbschalen- oder trogartig ausgebildeten Arbeitselemente schiebbar sind.

Weitere bevorzugte Ausführungsformen der Erfindung sind auch in den abhängigen Ansprüchen, der Beschreibung sowie der Zeichnung angegeben.

Die Erfindung wird im Folgenden beispielhaft unter Bezugnahme auf die Zeichnung beschrieben. Es zeigen:
- Fig. 1 - 4: verschiedene Ansichten einer Tiefenlehre gemäß einer Ausführungsform der Erfindung,
- Fig. 5: eine perspektivische Ansicht einer mit zwei erfindungsge-mäßen Tiefenlehren versehenen Probeeinheit gemäß einer Ausführungsform der Erfindung,
- Fig. 6: die Probeeinheit von Fig. 5 in einer Seitenansicht mit eingebrachtem Probekörper,
- Fig. 7: eine Tiefenlehre gemäß einer weiteren Ausführungsform der Erfindung,
- Fig. 8: die Tiefenlehre von Fig. 7 an einem Bestandteil einer erfindungsgemäßen Halteeinheit,
- Fig. 9 - 11: Bestandteile einer Bearbeitungseinheit gemäß einer Ausführungsform der Erfindung,
- Fig. 12: die Bestandteile von Fig. 9 - 11 im zusammengesetzten Zustand mit zwei erfindungsgemäßen Tiefenlehren,
- Fig. 13: eine Führungshülse der Bearbeitungseinrichtung von Fig. 12,
- Fig. 14: eine Bearbeitungseinheit gemäß einer weiteren Ausführungsform der Erfindung im zusammengesetzten Zustand mit zwei Tiefenlehren gemäß Fig. 7 und einem Bearbeitungsinstrument,
- Fig. 15: eine Ansicht von anterior der Bearbeitungseinheit von Fig. 14 ohne Bearbeitungsinstrument,
- Fig. 16: eine perspektivische Ansicht einer Halteeinheit gemäß einer weiteren Ausführungsform der Erfindung,
- Fig. 17: die Halteeinheit von Fig. 16 in einer Seitenansicht mit eingebrachten Implantatplatten,
- Fig. 18: einen Implantatkern eines mittels der Erfindung einsetzbaren Implantats mit einem Halteinstrument,
- Fig. 19: ein weiteres Halteinstrument für einen Implantatkern, und
- Fig. 20: auf einen Vorderteil einer lediglich teilweise dargestellten Distraktionszange aufgesteckte Verlängerungselemente zur Abstützung von Arbeitselementen der Bearbeitungseinheit von Fig. 14 und 15.

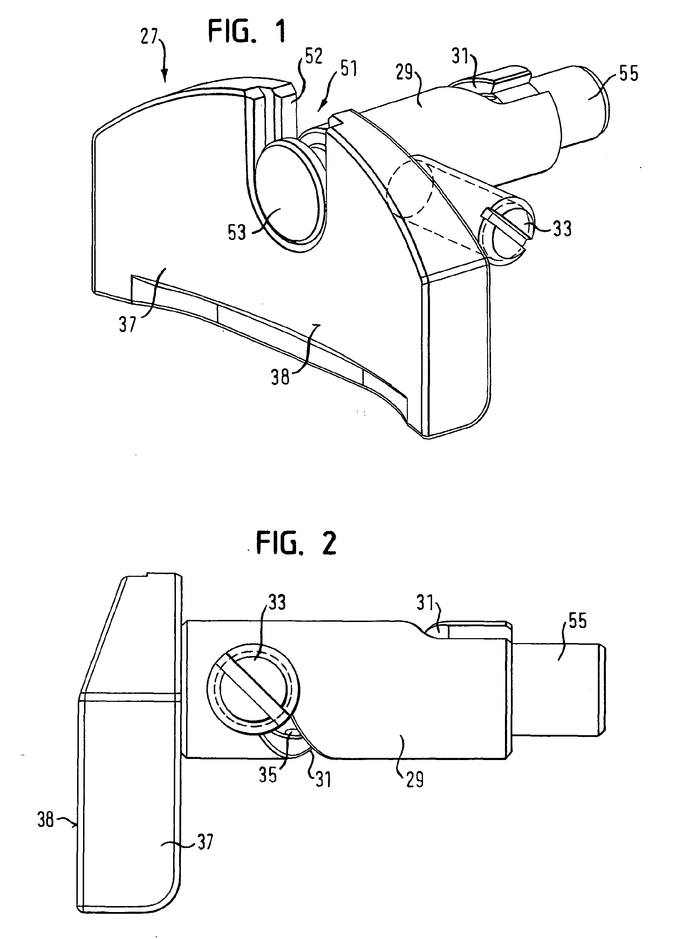

Die in den Fig. 1 bis 4 dargestellte erfindungsgemäße Tiefenlehre 27 umfasst eine Anschlagplatte 37 mit einer während der Operation senkrecht zur anterior-posterior-Richtung verlaufenden, nach posterior weisenden Anschlagfläche 38. Die Angabe "anterior-posterior" wird im Folgenden auch einfach mit "AP" abgekürzt.

Das Vorsehen einer derartigen Anschlagplatte ist erfindungsgemäß nicht zwingend. Alternativ kann die nach posterior weisende Stirnseite eines dem nachstehend beschriebenen Verstellabschnitt 29 entsprechenden, z.B. ebenfalls zylindrischen Verstellorgans als Anschlagfläche dienen und auf eine separate Anschlagplatte somit verzichtet werden.

Die Anschlagplatte 37 ist mit einer U-förmigen Aussparung 51 versehen, in die ein zylindrischer Verstellabschnitt 29 mit einem Verriegelungskopf 53 eingeführt ist. Im eingeführten Zustand hintergreift der scheibenförmige Verriegelungskopf 53 einen die Aussparung 51 bereichsweise begrenzenden Verriegelungsabschnitt 52. Hierdurch ist der Verstellzylinder 29 bezüglich der AP-Richtung fest mit der Anschlagplatte 37 verbunden, wobei jedoch die Lagerung des Verriegelungskopfes 53 in der Aussparung 51 ein Verdrehen des Verstellzylinders 29 gegenüber der Anschlagplatte 37 gestattet.

In der Außenwand des Verstellzylinders 29 ist eine wendelförmige Verstellnut 31 ausgebildet, in der während der Benutzung ein Führungsstift 33 mit einem Verstellfortsatz zwangsgeführt ist. Während der Benutzung ist der Führungsstift 33 an der jeweiligen Einheit, relativ zu welcher die Tiefenlehre 27 zu verstellen ist, bezüglich der AP-Richtung fest angeordnet, so dass eine Drehung des Verstellzylinders 29 durch die mit dem Stift 33 gekoppelte Nut 31 in eine Translationsbewegung in AP-Richtung umgesetzt wird.

Anstelle des Führungsstiftes 33 kann beispielsweise auch eine mit einem in die Verstellnut 31 hineinragenden Stift versehene Blattfeder vorgesehen sein, die in geeigneter Weise fest mit der jeweiligen Einheit verbunden ist.

Für die Drehbetätigung des Verstellzylinders 29 ist dieser mit einem nach anterior weisenden Betätigungsabschnitt 55 versehen, an welchem ein spezieller Schraubendreher angesetzt wird, auf den an anderer Stelle näher eingegangen wird.

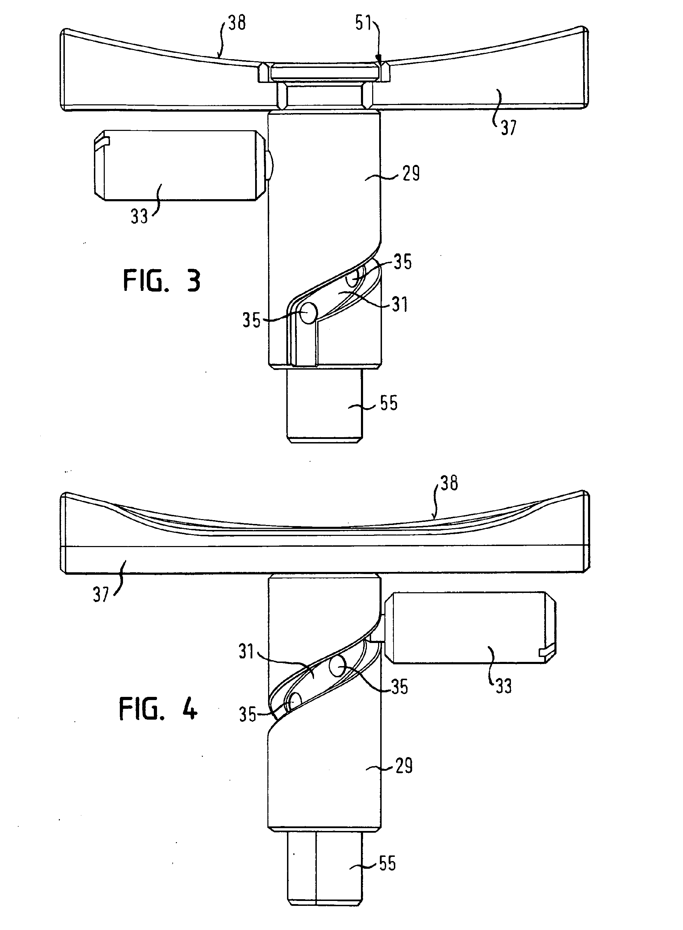

Der Seitenansicht in Fig. 2 ist insbesondere zu entnehmen, dass die Anschlagplatte 37 etwa im oberen Drittel, d.h. im Bereich der Aussparung 51 (Fig. 1), nach anterior abgeschrägt ist. Die Fig. 3 und 4 zeigen insbesondere, dass die Anschlagfläche 38 zur Anpassung an die Form der Wirbelkörper konkav gekrümmt ist.

Wie die Fig. 2, 3 und 4 zeigen, ist im Boden der Verstellnut 31 eine Mehrzahl von längs des Nutverlaufes beabstandet angeordneten Vertiefungen 35 ausgebildet. Der in die Verstellnut 31 hineinragende Verstellfortsatz des Führungsstiftes 33 ist in Richtung des Nutbodens mittels einer Druckfeder vorgespannt, so dass er in die Vertiefungen 35 einrastet, wenn die entsprechende Drehstellung des Verstellzylinders 29 erreicht ist. Die Verrastung ist derart ausgestaltet, dass die Raststellungen mittels des am Betätigungsabschnitt 55 angreifenden Schraubendrehers ohne zusätzliche Maßnahme übersteuert werden können, nichtsdestoweniger aber für den Operateur spürbar sind, so dass dem Operateur durch die Verrastungen das Erreichen der betreffenden Tiefenpositionen signalisiert wird.

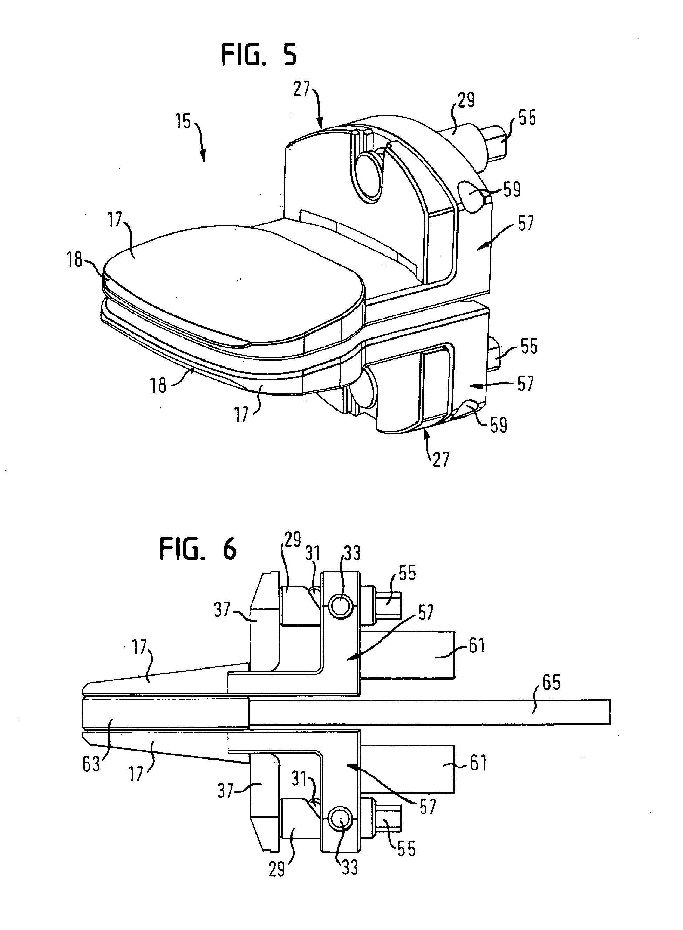

Die Fig. 5 und 6 zeigen zwei erfindungsgemäße Tiefenlehren 27 im an eine Probeeinheit 15 des erfindungsgemäßen Instrumentensystems angebrachten Zustand.

Die Probeeinheit 15 umfasst zwei im Wesentlichen baugleiche Winkelelemente 57. Der sich in AP-Richtung erstreckende Winkelabschnitt ist als plattenförmiger Probeabschnitt 17 ausgebildet. Die nach kaudal bzw. kranial weisende Seite der Probeplatte 17 ist entsprechend einem präoperativ im Rahmen der Operationsplanung bestimmten Winkel gegenüber der AP-Richtung geneigt. Für jede Implantatgröße steht ein Satz von Winkelelementen 57 mit unterschiedlichen Neigungen der Probeplatte 17 zur Verfügung.

Es ist festgestellt worden, dass es für die Praxis genügt, wenn sowohl für den oberen bzw. kranialen Teil als auch für den unteren bzw. kaudalen Teil der Probeeinheit 15 pro Implantatgröße lediglich zwei unterschiedlich stark geneigte Probeplatten 17 vorgehalten werden. Bei der einen Probeplatte beträgt der Neigungswinkel 2°, während der Neigungswinkel der anderen Probeplatte 7° beträgt. Die durch die möglichen Kombinationen von oberer und unterer Probeplatte 17 erreichbaren Gesamtwinkel zwischen den beiden geneigten Seiten der Probeplatten 17, nämlich Gesamtwinkel von 4°, 9° und 14°, haben sich als für die Praxis ausreichend erwiesen. Hierdurch kann die Anzahl der vorzuhaltenden Probeeinheiten 15 bzw. Winkelelemente 57 pro Implantatgröße überschaubar gehalten werden.

Im Bereich ihrer nach kranial bzw. kaudal weisenden posterioren Vorderkante sind die Probeplatten 17 mit einer Abflachung 18 versehen, die das Einführen in das Bandscheibenfach vereinfacht. In dem senkrecht zur AP-Richtung verlaufenden Abschnitt der Winkelelemente 57 sind eine in AP-Richtung verlaufende Bohrung für den Verstellzylinder 29 der Tiefenlehre 27 sowie eine senkrecht dazu verlaufende Bohrung 59 für den in Fig. 5 nicht dargestellten Führungsstift 33 ausgebildet. Der Führungsstift 33 wird in die Bohrung 59 eingeschraubt und ist hierzu mit einem Schlitz versehen (Fig. 2).

Während die Bestimmung der Implantatgröße sowie der Implantatneigung präoperativ erfolgt und zur Auswahl der passenden Probeplatten 17 führt, erfolgt die Bestimmung der passenden Implantathöhe oder -dicke intraoperativ bei in das Bandscheibenfach eingeführten Probeplatten 17. Hierzu ist ein Satz von unterschiedliche Dicken aufweisenden, jeweils an einer Haltestange 65 angebrachten Probekörpern 63 vorgesehen, die bei der Operation zwischen die entsprechend auseinander gedrückten Probeplatten 17 eingebracht werden. Die passende Implantathöhe ist gefunden, wenn der intraoperativ kontrollierbare Verlauf der Wirbelsäule des Patienten bzw. die Stellung der betreffenden Wirbelkörper der natürlichen Vorgabe am besten entspricht.

Die Auswahl der Implantathöhe mittels der Probekörper 63 und damit die abschließende Auswahl des einzusetzenden Implantats erfolgt hierbei nicht nur unter Berücksichtigung der präoperativ bestimmten, durch die geneigten Probeplatten 17 "simulierten" Winkelvorgabe, sondern außerdem unter Berücksichtigung der ebenfalls präoperativ bestimmten, für den betreffenden Patienten optimalen Einsetztiefe des Implantats, d.h. dessen AP-Position, die an der Probeeinheit 15 mittels der Tiefenlehren 27 als Referenz für die nachfolgenden Operationsschritte eingestellt wird. Die Einhaltung der präoperativ ausgewählten AP-Position des Implantats wird also durch die erfindungsgemäßen Tiefenlehren 27 sichergestellt.

Wie Fig. 6 zeigt, reichen die Anschlagplatten 37 jeweils bis zum in AP-Richtung verlaufenden Abschnitt des Winkelelements 57. Die Probeplatten 17 bilden so einen posterioren Endanschlag für die Anschlagplatten 37, wobei dies aber nicht zwingend erforderlich ist, sondern alternativ oder zusätzlich z.B. ein solcher Endanschlag auch durch einen anterioren Endanschlag der Verstellnut 31 erreicht werden könnte.

Zum Auseinanderdrücken der Probeplatten 17 sind an den senkrecht zur AP-Richtung verlaufenden Abschnitten der Winkelelemente 57 nach anterior vorstehende Kopplungsabschnitte 61 ausgebildet. Auf die Kopplungsabschnitte 61 können die beiden Backen einer Distraktionszange aufgesteckt werden. Die Kopplungsabschnitte 61 sind hierzu an die Form der Zange angepasst. Es können bekannte, im Handel frei erhältliche Distraktionszangen verwendet werden.

Die Kopplungsabschnitte 61 sind seitlich aus der Mitte versetzt angeordnet, so dass beim Einschieben der Probekörper 63 mittels der Haltestangen 65 die Zange nicht im Wege ist.

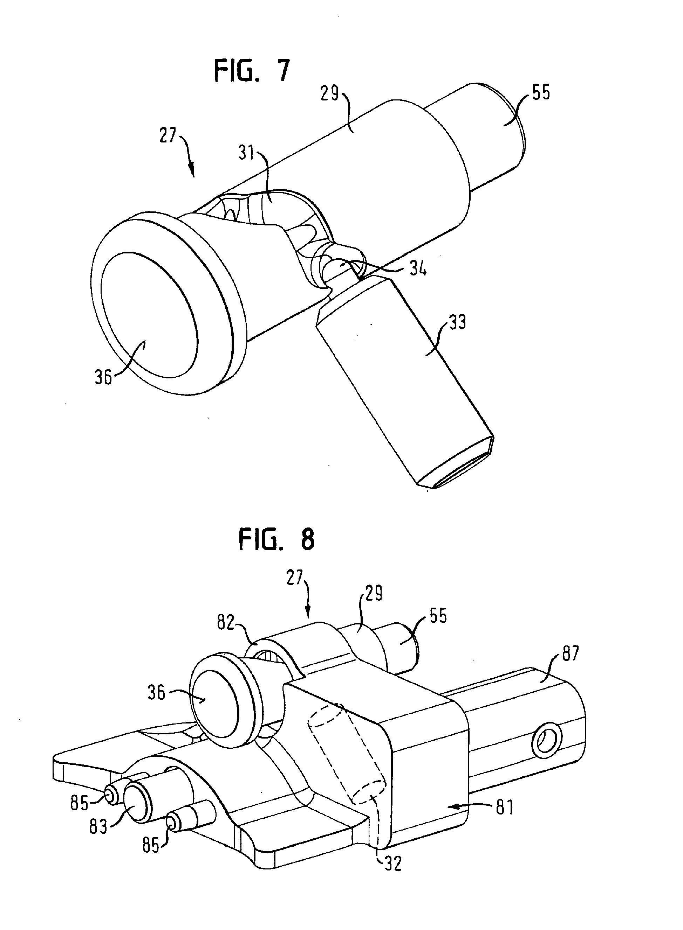

Die in Fig. 7 dargestellte Tiefenlehre 27 weist keine Anschlagplatte auf. Als Anschlagfläche 36 dient die nach posterior weisende Stirnfläche 36 des scheibenförmigen Kopfes des zylindrischen Verstellabschnitts 29. Ein weiterer Unterschied zu dem Ausführungsbeispiel der Fig. 1 bis 6 besteht in dem Verlauf der Verstellnut 31 für den am Führungsstift 33 ausgebildeten Vorsprung 34.

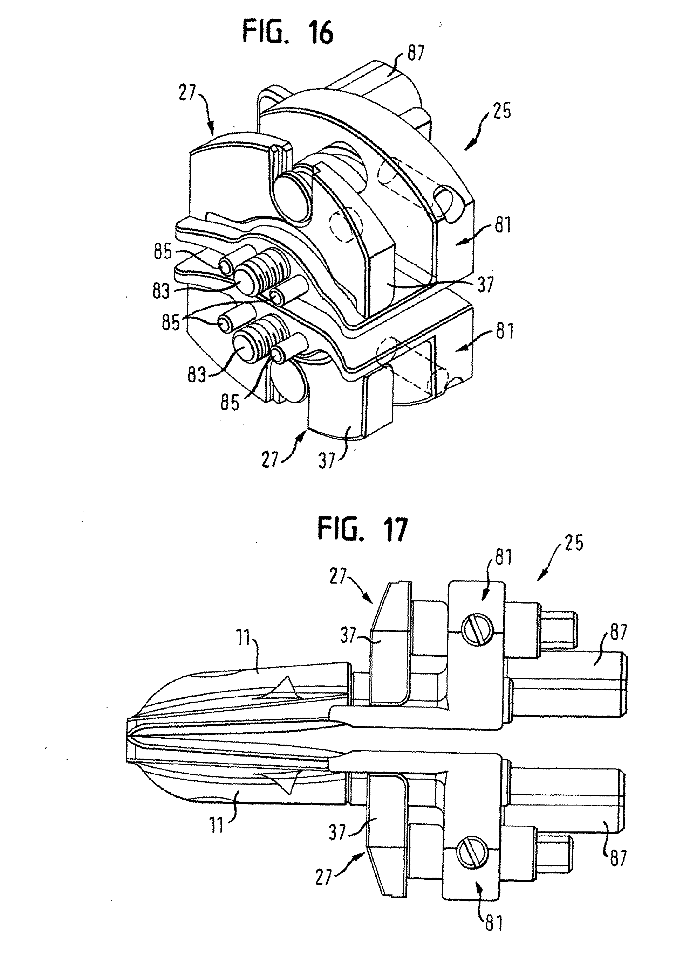

Fig. 8 zeigt die Tiefenlehre 27 an einer Hälfte einer Halteeinheit. Auf den prinzipiellen Aufbau einer erfindungsgemäßen Halteeinheit wird nachstehend in Verbindung mit den Fig. 16 und 17 näher eingegangen. Anders als bei der dort dargestellten Ausführungsform sind die beiden Hälften der Halteeinheit gemäß Fig. 8 mit einem Ringabschnitt 82 zur Durchführung des Verstellabschnitts 29 der Tiefenlehre 27 versehen. Der Ringabschnitt 82 ist an einem Winkelelement 81 ausgebildet, das außerdem mit einer Halteschraube 83 und Positionierungsstiften 85 zum Halten der hier nicht dargestellten Implantatplatten sowie einem Kopplungsabschnitt für ein Distraktionsinstrument versehen ist. Hierauf wird in Verbindung mit den Fig. 16 und 17 näher eingegangen.

Die Tiefenlehre 27 ist in Fig. 8 ohne den Führungsstift 33 (Fig. 7) dargestellt. Eine im Winkelelement 81 ausgebildete Bohrung 32 für den Führungsstift 33 ist in Fig. 8 durch gestrichelte Linien angedeutet.

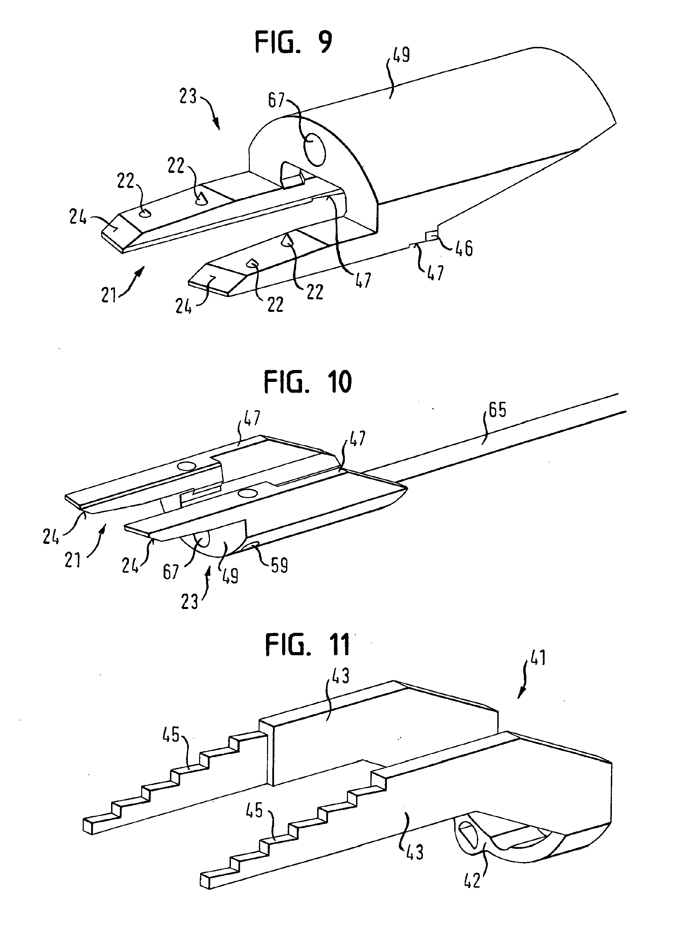

Die Fig. 9 bis 13 zeigen eine erfindungsgemäße Bearbeitungseinheit 19, die zur Vorbereitung der Wirbelkörperflächen verwendet wird, sobald die passende Implantatgröße bestimmt ist.

Die Bearbeitungseinheit 19 umfasst zwei unterschiedlich ausgebildete Arbeitselemente 23. Das in Fig. 9 gezeigte Arbeitselement 23 wird im Folgenden auch als oberes oder kraniales und das Arbeitselement 23 gemäß Fig. 10 als unteres oder kaudales Arbeitselement bezeichnet, obwohl prinzipiell die Bearbeitungseinheit 19 auch in umgekehrter Orientierung verwendet werden kann.

Die Arbeitselemente 23 umfassen jeweils einen halbschalen- oder trogförmigen Körperabschnitt 49, von dem nach posterior ein gabelförmiger Abschnitt 21 mit zwei in AP-Richtung verlaufenden Gabelarmen absteht. Zur Erleichterung des Einführens der Gabelabschnitte 21 in den Zwischenwirbelraum des Patienten sind die freien Enden der Gabelarme jeweils mit einer Abflachung 24 versehen. Um der Bearbeitungseinheit 19 während des Bearbeitungsvorgangs einen möglichst sicheren Halt an den Wirbelkörperflächen zu verleihen, sind die Gabelarme an der entsprechenden Seite jeweils mit Haltevorsprüngen 22 versehen.

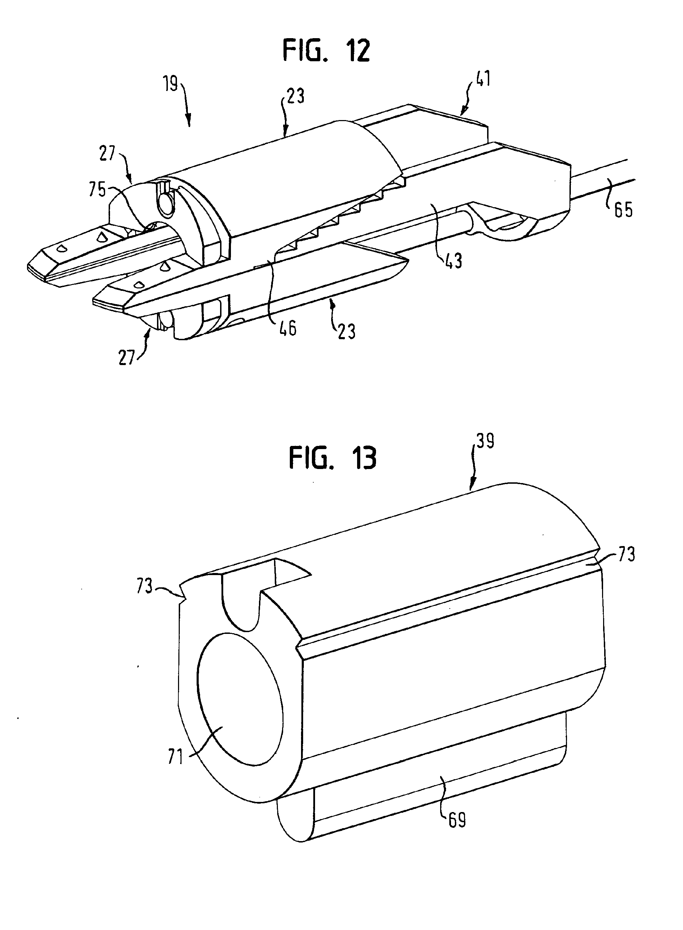

Zur Anbringung der erfindungsgemäßen Tiefenlehre 27 (Fig. 12) sind die beiden Arbeitselemente 23 jeweils mit einer Bohrung 67 für den Verstellzylinder 29 der Tiefenlehre 27 und mit einer Bohrung 59 (in Fig. 9 nicht dargestellt) für den Führungsstift 33 versehen.

An ihrer offenen Seite weisen die Arbeitselemente 23 jeweils Längskantenabschnitte 47 auf, über welche die Arbeitselemente 23 mit einem nachstehend näher erläuterten Verriegelungselement 41 (Fig. 11) zusammenwirken. Am kranialen Arbeitselement 23 (Fig. 9) ist dieser Längskantenabschnitt 47 an einer Stufe 46 ausgebildet.

Während der Operation dem Zwischenwirbelfach zugeführt werden die Arbeitselemente 23 mittels Haltestangen 65. In Fig. 10 ist eine solche Haltestange 65 in Verbindung mit dem unteren bzw. kaudalen Arbeitselement 23 dargestellt.

Das erfindungsgemäße Verriegelungselement 41 gemäß Fig. 11 umfasst einen U-förmigen Körperabschnitt 42, mit welchem es längs der Außenseiten der Arbeitselemente 23 in AP-Richtung geführt werden kann. Die U-Schenkel des Körperabschnitts 42 erweitern sich zu in AP-Richtung verlaufenden Verriegelungsarmen 43, die an ihren der offenen Seite des Körperabschnitts 42 zugewandten Längskanten mit einem gestuften bzw. treppenförmigen Höhenprofil 45 versehen sind.

Im zusammengesetzten Zustand gemäß Fig. 12 sind die Arbeitselemente 23 jeweils mit einer Tiefenlehre 27 versehen. Die mit der Probeeinheit 15 (Fig. 5) ermittelte Referenzeinstellung der Tiefenlehren 27 wird entsprechend an den Tiefenlehren 27 der Bearbeitungseinheit 19 eingestellt, bevor diese mit den gabelförmigen Arbeitsabschnitten 21 in den Zwischenwirbelraum eingeführt wird. Hierbei befindet sich das Verriegelungselement 41 bereits in der in Fig. 12 dargestellten Ausgangsposition, in welcher die Verriegelungsarme 43 stirnseitig auf den Längskantenabschnitten 47 des unteren Arbeitselementes 23 aufliegen und an den Stufen 46 des oberen Arbeitselementes 23 anliegen.

Die beiden halbschalen- bzw. trogförmigen Körperabschnitte 49 der Arbeitselemente 23 bilden einen tunnelartigen Aufnahmeraum. In diesen Aufnahmeraum wird zwischen den Verriegelungsarmen 43 hindurch eine Führungshülse 39 (Fig. 13) für ein nicht dargestelltes Bearbeitungsinstrument, insbesondere in Form eines Fräsers, eingeführt. Die Führungshülse 39 ist auf einer Seite mit einem Paar von Führungsnuten 73 versehen, denen sowohl an der Innenseite des oberen Arbeitselementes 23 als auch an der Innenseite des unteren Arbeitselementes 23 ausgebildete Führungselemente (nicht dargestellt) zugeordnet sind.

Durch diese Führung ist die Fräshülse 39 beim Einführen in den von den beiden Arbeitselementen 23 gebildeten Aufnahmeraum ausschließlich längs einer durch die Führung vorgegebenen Bearbeitungsachse relativ zum betreffenden Arbeitselement 23 bewegbar und in Richtungen senkrecht zu dieser Bearbeitungsachse am Arbeitselement 23 unbeweglich gehalten. Die Richtung der Bearbeitungsachse kann, muss aber nicht mit der AP-Richtung zusammenfallen. Vielmehr ist es bevorzugt, wenn die durch die Führung vorgegebene Bearbeitungsachse einen Bearbeitungswinkel mit der AP-Richtung einschließt, der dem Neigungswinkel der Probeplatten 17 bzw. der Implantatplatten 11 entspricht. Da dieser Bearbeitungswinkel vergleichsweise klein ist, verläuft die Bearbeitungsachse auch in diesem Fall immer noch näherungsweise in AP-Richtung.

Durch die Führung der Fräshülse 39 ist senkrecht zur Bearbeitungsachse, insbesondere also näherungsweise in Distraktions- oder Aufspreizrichtung, eine eindeutig festgelegte Höhe der Mittelachse einer für den Fräser vorgesehenen Durchgangsbohrung 71 relativ zu dem Gabelabschnitt 21 und damit zu dem betreffenden Wirbelkörper vorgegeben.

Die Bearbeitung der betreffenden Wirbelkörperfläche mittels des durch die Bohrung 71 hindurch geführten Fräsers erfolgt somit in einer durch die Distraktion eindeutig vorgegebenen Höhe. Die Bearbeitungstiefe ist durch die zuvor auf die Referenzposition eingestellten Tiefenlehren 27 festgelegt, die wiederum auf eine Referenz für die Einführtiefe der Hülse 39 bzw. des Fräsers abgestimmt sind. Die Arbeitselemente 23 oder die Hülse 39 können Endanschläge für den Fräser aufweisen.

Bereits vor dem Einführen des Fräsers in die Führungshülse 39 stehen somit alle Parameter der Wirbelkörperbearbeitung fest, was die Vorbereitung der Wirbelkörperflächen für den Operateur erheblich vereinfacht.

Anders als bei den in Verbindung mit der Probeeinheit 15 (Fig. 5) verwendeten Tiefenlehren 27 sind die Anschlagplatten 38 der in Verbindung mit der Bearbeitungseinheit 19 (Fig. 12) verwendeten Tiefenlehren 27 jeweils mit einer Aussparung 75 für den durch die Bohrung 71 der Führungshülse 39 hindurchgeführten Fräser versehen.

Um im Anschluss an die Vorbereitung der einen Wirbelkörperfläche den anderen Wirbelkörper zu bearbeiten, braucht die Führungshülse 39 lediglich aus dem Aufnahmeraum herausgezogen, um 180° um eine in AP-Richtung verlaufende Achse gedreht und auf das andere Arbeitselement 23 aufgeschoben zu werden.

Die Bearbeitung der Wirbelkörper mittels des durch die Hülse 39 geführten Fräsers erfolgt bei einem mit Hilfe der Distraktionszange eingestellten Abstand zwischen den beiden Arbeitselementen 23. Hierzu sind nicht dargestellte Adapterelemente vorgesehen, die auf die Backen der Distraktionszange aufgesteckt werden, um die Distraktionszange an entsprechend den Adapterelementen ausgebildete Angriffsbereiche (nicht dargestellt) an den Innenseiten der Arbeitselemente 23 anzupassen. Die Adapterelemente sind insofern selbstverriegelnd ausgebildet, als sie mit speziellen Hinterschneidungen versehen sind, die im an die Arbeitselemente 23 angesetzten Zustand ein versehentliches Lösen der Distraktionszange von den Arbeitselementen 23 sicher verhindern. Separate Verriegelungseinrichtungen sind hierdurch nicht erforderlich.

Während des Aufspreizvorgangs wird das Verriegelungselement 41 schrittweise weiter zwischen die Arbeitselemente 23 geschoben. Die den gestuften Höhenprofilen 45 gegenüberliegenden, gerade ausgeführten Längskanten der Verriegelungsarme 43 liegen dabei auf den Längskantenabschnitten 47 des unteren Arbeitselementes 23 auf, während die am oberen Arbeitselement 23 ausgebildeten Stufen 46 mit den Treppenprofilen 45 der Verriegelungsarme 43 zusammenwirken. Folglich kann durch schrittweises Einschieben des Verriegelungselementes 41 eine einmal erreichte Distraktionshöhe gesichert, d.h. die Bearbeitungseinheit 19 in dieser Stellung verriegelt werden. Sobald die gewünschte, in Verbindung mit der Probeeinheit 15 (Fig. 5) bestimmte und dem passenden Probekörper 63 (Fig. 6) entsprechende Distraktionshöhe erreicht und mittels des Verriegelungselementes 41 gesichert ist, kann die Distraktionszange einschließlich der Adapterelemente entfernt werden. Der von den Arbeitselementen 23 gebildete Tunnel ist nunmehr frei zur Aufnahme der Führungshülse 39.

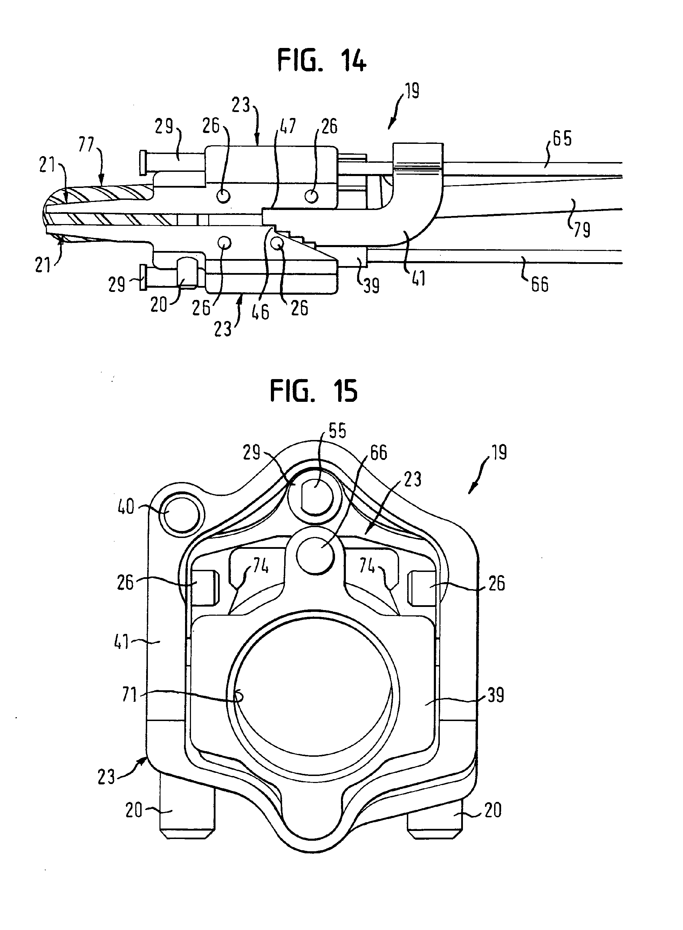

Das in Fig. 14 und 15 dargestellte weitere Ausführungsbeispiel einer erfindungsgemäßen Bearbeitungseinheit 19 umfasst zwei Arbeitselemente 23, die jeweils mit einer Tiefenlehre gemäß Fig. 7 versehen sind, von welcher der Verstellzylinder 29 und (Fig. 15) der Betätigungsabschnitt 55 dargestellt sind. In Aufspreizrichtung, d.h. senkrecht zur AP-Richtung, relativ zueinander geführt sind die Arbeitselemente 23 durch Stifte 20.

Ferner ist in Fig. 14 ein - in Fig. 15 weggelassenes -Bearbeitungsinstrument 77 in Form eines Fräsers dargestellt, das durch die in Fig. 14 mit dem oberen und in Fig. 15 mit dem unteren Arbeitselement 23 gekoppelte Führungshülse 39 geführt wird. Aus Fig. 14 geht insbesondere der vorstehend bereits erwähnte, von Null verschiedene Bearbeitungswinkel zwischen der Haltestange 79 und Drehachse des Fräsers und der theoretischen AP-Richtung hervor, parallel zu welcher sich die in Fig. 14 dargestellte Haltestange 65 des oberen Arbeitselementes 23 erstreckt. Eine Haltestange 66 der Führungshülse 39 verläuft bei eingeschobenem Fräser 77 geneigt zu dessen Haltestange 79.

Das mit den Stufen 46 bzw. den Längskantenabschnitten 47 der Arbeitselemente 23 zusammenwirkende Verriegelungselement 41 kann über eine nicht dargestellte Haltestange gehandhabt werden, für die es mit einer seitlich nach außen versetzt angeordneten Öffnung 40 (Fig. 15) versehen ist.

Die mit der Bohrung 71 für den Fräser 77 versehene Führungshülse 39 ist zunächst mit dem einen und dann mit dem anderen Arbeitselement 23 koppelbar, um nacheinander die beiden Wirbelkörper zu bearbeiten. Hierzu sind die Arbeitselemente 23 mit in Fig. 15 nur am oberen Arbeitselement 23 erkennbaren Führungsstiften 26 für entsprechende Führungsnuten der Führungshülse 39 versehen. Des Weiteren sind die Arbeitselemente 23 mit einer rinnenartigen Vertiefung zur Führung der entsprechend geformten, dem Abschnitt für die Haltestange 66 gegenüberliegenden Seite der Führungshülse 39 versehen.

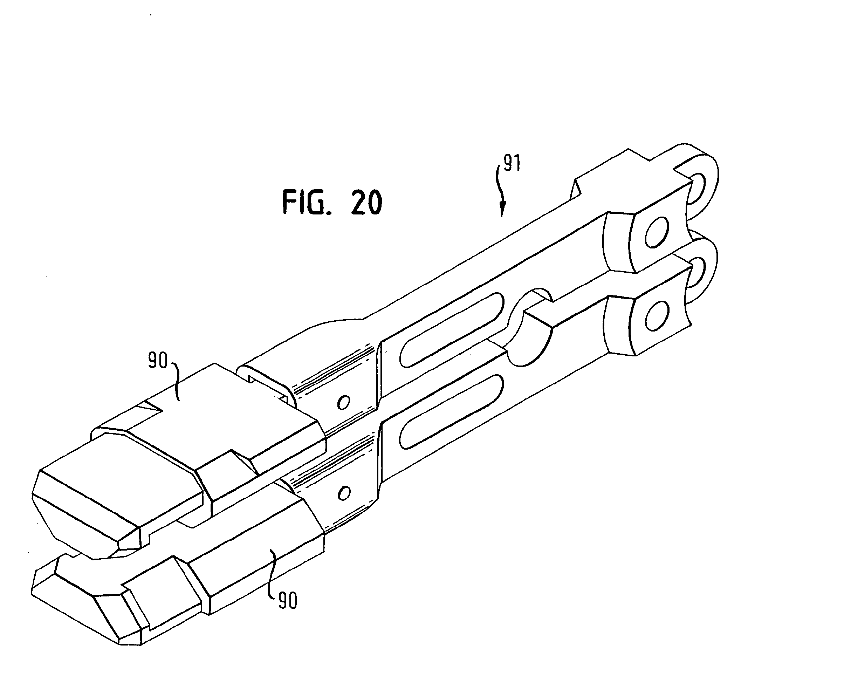

Ferner sind die Arbeitselemente 23 jeweils mit innen liegenden Schultern 74 versehen, über welche sich die Arbeitselemente 23 an in Fig. 20 dargestellten Adapter- oder Verlängerungselementen 90 abstützen können, die auf die vorderen Enden einer Distraktionszange 91 aufgesteckt sind.

Die in den Fig. 16 und 17 dargestellte Halteeinheit 25 entspricht hinsichtlich ihres prinzipiellen Aufbaus der Probeeinheit 15 (Fig. 5). Die sich in AP-Richtung erstreckenden Abschnitte der Winkelelemente 81 sind jeweils mit Positionierungsstiften 85 sowie mit einer zwischen den Stiften 85 gelegenen Durchgangsbohrung für eine separate Halteschraube 83 versehen. Mittels der Halteschrauben 83 ist die jeweilige Implantatplatte 11 (Fig. 17) beim Einsetzen in den Zwischenwirbelraum in der durch die Positionierungsstifte 85 festgelegten Lage relativ zum Winkelelement 81 fest mit diesem verbunden. An ihren anterioren Rückseiten sind die Implantatplatten 11 jeweils mit entsprechenden Einstecköffnungen für die Positionierungsstifte 85 sowie mit einer Gewindebohrung für die Halteschraube 83 versehen.

Anstelle der in Fig. 16 dargestellten, rein zylindrischen Positionierungsstifte 85 können auch Positionierungsstifte mit anders geformten freien Enden verwendet werden. Beispielsweise können die freien Endabschnitte der Positionierungsstifte 85 spitzkegelig zulaufen oder kegelstumpfförmig ausgebildet sein, wobei auch die Länge dieser nicht-zylindrischen Endabschnitte der Stifte variieren kann. Alternativ zu dieser Stift/Loch-Positionierung kann auch eine so genannte Aufsitz-Positionierung vorgesehen sein, bei welcher anstelle der Stifte 85 ein oder mehrere, beispielsweise schippenartig ausgebildete Trägervorsprünge angeordnet sind, auf welchen die mittels der Halteschrauben 83 befestigten Implantatplatten 11 aufsitzen. Auch auf diese Weise lässt sich eine sichere Positionierung der einzusetzenden Implantatplatten 11 erzielen.

Die Querschnittsflächen der parallel zur AP-Richtung verlaufenden Abschnitte der Winkelelemente 81 sind jeweils entsprechend der anterioren Rückseite der Implantatplatte 11 geformt und dementsprechend mit einer mittigen konvexen Überhöhung versehen, durch welche die Durchgangsbohrung für die Halteschraube 83 verläuft. Die Anschlagplatten 37 der Tiefenlehren 27 sind daher mit einer entsprechenden Aussparung versehen.

Fig. 13 zeigt die erfindungsgemäße Halteeinheit 25 mit daran angebrachten, mittels der Halteschrauben 83 (Fig. 16) fixierten Implantatplatten 11. Die richtige, der Implantatauswahl entsprechende Einsetztiefe für die Implantatplatten 11 wird wiederum durch die erfindungsgemäßen Tiefenlehren 27 sichergestellt, an denen die mit den Tiefenlehren 27 der Probeeinheit (Fig. 5) zuvor ermittelte Referenzposition eingestellt ist.

Fig. 17 zeigt den optimalen Fall, in welchem die Tiefenlehren 27 derart eingestellt sind, dass die hintere anteriore Kante der Implantatplatten 11 etwa 4 mm hinter - von anterior nach posterior gesehen - der anterioren Kante der nicht dargestellten Wirbelkörper liegt. Je nach Einstellung der Tiefenlehren 27, d.h. in Abhängigkeit von dem Abstand zwischen den Anschlagplatten 37 und den senkrecht zur AP-Richtung verlaufenden Abschnitten der Winkelelemente 81, liegt die anteriore Kante der Implantatplatten 11 mehr oder weniger weit von der anterioren Wirbelkörperkante entfernt.

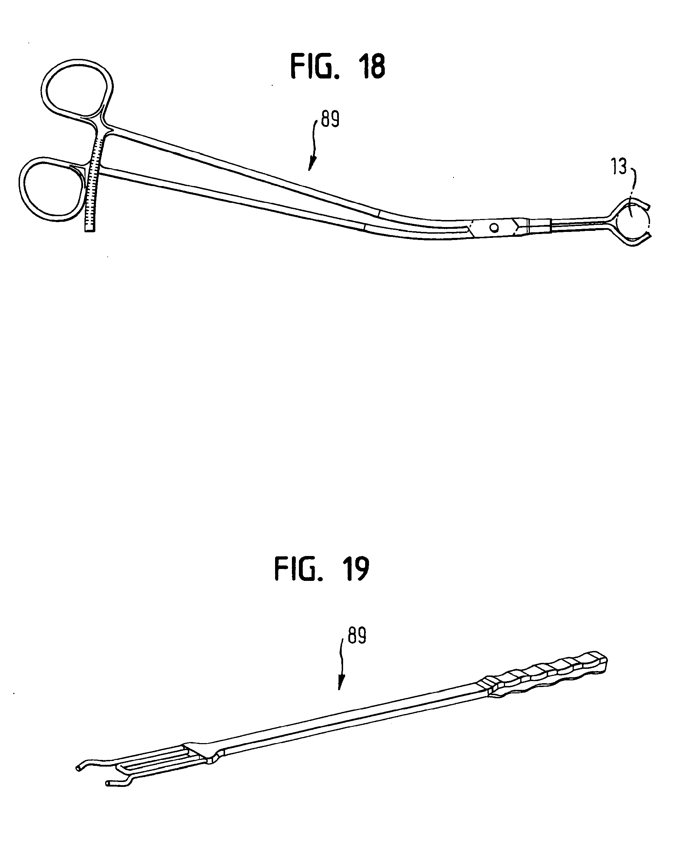

Über entsprechend der Probeeinheit 15 (Fig. 5) außermittig versetzt angeordnete Kopplungsabschnitte 87 werden die Winkelelemente 81 mit der Distraktionszange gekoppelt. Sobald sich die in den Zwischenwirbelraum eingeführten Implantatplatten 11 in der Solltiefe befinden und durch Betätigen der Distraktionszange die Wirbelkörper ausreichend weit aufgespreizt worden sind, wird der passende, vorzugsweise linsenförmig ausgebildete Implantatkern 13 (Fig. 18), auf den nicht näher eingegangen werden soll, zwischen die Implantatplatten 11 gebracht. Hierzu ist eine Haltezange 89 vorgesehen. In Fig. 18 dargestellt ist eine Haltezange 89 vom so genannten Krebs-Typ. Alternativ kann beispielsweise auch eine Haltezange vom so genannten Nadelhalter-Typ Verwendung finden, wie sie beispielhaft in Fig. 19 dargestellt ist.

Sobald sich der auch als Inlay bezeichnete Implantatkern 13 in der Sollposition zwischen den beiden distrahierten Implantatplatten 11 befindet, wird die Distraktion beendet, d.h. die Distraktionszange gewissermaßen "losgelassen". Das aus den beiden Implantatplatten 11 und dem Implantatkern 13 bestehende Bandscheibenimplantat gelangt so in seine Endkonfiguration. Um die Winkelabschnitte 81 der Halteeinheit 25 von den Implantatplatten 11 zu lösen, werden die Halteschrauben 83 mittels eines Schraubendrehers gelöst und die Winkelabschnitte 81 mitsamt den Positionierungsstiften 85 zurückgezogen. Anschließend wird die Halteeinheit 25 ausgefahren. Die Halteschrauben 83 sind von der anterioren Seite aus mittels des Schraubendrehers zugänglich, wobei die seitlich aus der Mitte versetzte Anordnung der Kopplungsabschnitte 87 für die Distraktionszange nicht nur das Einsetzen des Implantatkerns 13 mittels der Haltezange 89, sondern auch das Lösen der Halteschrauben 83 erleichtert.

Der Ablauf einer mittels des erfindungsgemäßen Instrumentensystems durchgeführten Operation zum Einsetzen eines zwei Implantatplatten 11 und einen Implantatkern 13 umfassenden Bandscheibenimplantats ergibt sich bereits aus der vorstehenden Beschreibung des erfindungsgemäßen Instrumentariums. Nachstehend wird noch auf besondere Aspekte der Erfindung eingegangen und das erfindungsgemäße Konzept noch einmal im Zusammenhang dargestellt.

Wie vorstehend bereits erwähnt, werden in der ersten, der präoperativen Operationsphase zunächst die Größe des Implantats sowie der Neigungswinkel der Implantatplatten 11 bestimmt, also der Neigungswinkel der im eingesetzten Zustand den Wirbelkörperflächen zugewandten Seiten der Implantatplatten 11 relativ zur AP-Richtung. Ferner wird in dieser präoperativen Phase die optimale Lage des Implantats in AP-Richtung, also die Einsetztiefe, bestimmt. Diese präoperative Festlegung von Operationsparametern erfolgt mit Hilfe von Bildmaterial, das durch unterschiedliche, grundsätzlich bekannte Verfahren (z.B. Röntgenstrahlung, MRI, CT) gewonnen werden kann, worauf an dieser Stelle nicht näher eingegangen werden soll.

Für die anschließende intraoperative Parameterbestimmung wird die zuvor ermittelte AP-Position mittels eines speziellen Schraubendrehers an den Tiefenlehren 27 der Probeeinheit 15 (Fig. 5) eingestellt. Es wird eine Probeeinheit 15 verwendet, deren Probeplatten 17 sowohl hinsichtlich der präoperativ bestimmten Implantatgröße als auch hinsichtlich des präoperativ bestimmten Neigungswinkels dem vorausgewählten Implantat entspricht. Dabei wird aber so vorgegangen, dass man sich unter Bildschirmkontrolle schrittweise an die präoperativ bestimmte Stellung annähert.

Der spezielle Schraubendreher, mit welchem die AP-Einstellung an den Tiefenlehren 27 vorgenommen wird, zeichnet sich durch eine Anzeigeeinrichtung aus, an welcher die vorgenommene AP-Einstellung abgelesen und so für die nachfolgenden AP-Einstellungen als Referenz verwendet werden kann. Hierzu kann in einem bevorzugten Ausführungsbeispiel der Schraubendreher mit einer Skalenscheibe versehen sein, die drehfest mit dem Schraubendreherschaft in der Nähe des Griffes angeordnet ist. Durch die von einer reinen Kreiszylinderform abweichende Ausgestaltung der Betätigungsabschnitte 55 (Fig. 4, Fig. 5 und Fig. 6) der Tiefenlehren 27 ist eine eindeutige relative Rotationslage zwischen Schraubendreherschaft und Verstellzylinder 29 der Tiefenlehre 27 gegeben. Die an der Probeeinheit 15 vorgenommene AP-Einstellung kann folglich am Schraubendreher abgelesen und korrekt auf die Tiefenlehren 27 der Bearbeitungseinheit 19 sowie der Halteeinheit 25 übertragen werden.

Die bei der Auswahl des passenden Probekörpers 63 (Fig. 6) zum Aufspreizen der Probeeinheit 15 verwendete Distraktionszange verfügt ebenfalls über die Möglichkeit, eine als passend ausgewählte Distraktionshöhe abzulesen und reproduzierbar bei nachfolgenden Distraktionsvorgängen erneut einzustellen. Im Anschluss an die intraoperativ erfolgende Bestimmung der passenden Implantathöhe mittels der Probekörper 63 verfügt der Operateur folglich sowohl über einen - präoperativ bestimmten - Referenzwert für die AP-Einstellung als auch über einen - intraoperativ mittels der Probeeinheit 15 ermittelten - Referenzwert für die Distraktionshöhe.

Die anschließende Vorbereitung der Wirbelkörperflächen mittels der Bearbeitungseinheit 19 (Fig. 12 und 13) erfolgt somit in einer Stellung der maßgeblichen, für die Führung des Fräsintrumentes dienenden Führungshülse 39 relativ zu dem betreffenden Wirbelkörper, die durch die AP-Einstellung an den Tiefenlehren 27 der Bearbeitungseinheit 19 und durch die zuvor bestimmte Distraktionshöhe eindeutig festgelegt ist. Die Bearbeitung der Wirbelkörper wird folglich in einer gezielt auf das ausgewählte Implantat abgestimmten und für den Operateur äußerst komfortablen Weise durchgeführt.

Im Anschluss an die Vorbereitung der Wirbelkörperflächen werden die Implantatplatten 11 des Implantats mittels der Halteeinheit 25 eingesetzt, wobei wiederum die entsprechend dem zuvor ermittelten Referenzwert eingestellten Tiefenlehren 27 für die korrekte AP-Position und damit für die richtige Einsetztiefe der Implantatplatten 11 und damit des Implantats insgesamt sorgen.

Durch die Erfindung wird dem Operateur ein einfach handhabbares, eine überschaubare Anzahl von Einzelteilen umfassendes Instrumentarium an die Hand gegeben, mit welchem Bandscheibenoperationen mit einem hohen Maß an Genauigkeit und Sicherheit durchgeführt werden können.

### Bezugszeichenliste

- 11: Implantatplatte
- 13: Implantatkern
- 15: Probeeinheit
- 17: Probeabschnitt, Probeplatte
- 18: Abflachung
- 19: Bearbeitungseinheit
- 20: Stift
- 21: gabelförmiger Abschnitt des Arbeitselementes
- 22: Haltevorsprung
- 23: Arbeitselement
- 24: Abflachung
- 25: Halteeinheit
- 26: Führungsstift
- 27: Tiefenlehre
- 29: Verstellabschnitt der Tiefenlehre, Verstellzylinder
- 31: Verstellnut
- 32: Bohrung
- 33: Führungsstift
- 34: Vorsprung
- 35: Rastvertiefung
- 36: Anschlagfläche
- 37: Anschlagplatte
- 38: Anschlagfläche
- 39: Führungshülse
- 40: Öffnung
- 41: Verriegelungselement
- 42: Körperabschnitt
- 43: Verriegelungsarm
- 45: Höhenprofil
- 46: Stufe
- 47: Längskantenabschnitt
- 49: Körperabschnitt
- 51: Aussparung der Anschlagplatte
- 52: Verriegelungsabschnitt
- 53: Verriegelungskopf
- 55: Betätigungsabschnitt
- 57: Winkelelement
- 59: Bohrung
- 61: Kopplungsabschnitt
- 63: Probekörper
- 65: Haltestange
- 66: Haltestange
- 67: Bohrung für Tiefenlehre
- 69: Halteabschnitt
- 71: Bohrung
- 73: Führungsnut
- 74: Schulter
- 75: Aussparung
- 77: Bearbeitungsinstrument
- 79: Haltestange
- 81: Winkelelement
- 82: Ringabschnitt
- 83: Halteschraube
- 85: Positionierungsstift
- 87: Kopplungsabschnitt
- 89: Haltezange
- 90: Verlängerungselement
- 91: Distraktionsinstrument

## Patentansprüche

1. Instrumentensystem zum Einsetzen von zwei Implantatplatten (11) und einen Implantatkern (13) umfassenden Bandscheibenimplantaten, mit
- wenigstens einer zwei vorzugsweise plattenförmige Probeabschnitte (17) umfassenden Probeeinheit (15) zum Bestimmen eines für den jeweiligen Patienten passenden Implantats,
- zumindest einer Bearbeitungseinheit (19) zum Vorbereiten der Wirbelkörperflächen des Patienten, die zwei vorzugsweise gabelförmige Abschnitte (21) aufweisende Arbeitselemente (23) umfasst, und
- wenigstens einer mit den Implantatplatten (11) koppelbaren Halteeinheit (25) zum Einsetzen des Implantats,
wobei die Probeeinheit (15), die Bearbeitungseinheit (19) und die Halteeinheit (25) zum Auseinanderdrücken der Probeabschnitte (17), der Arbeitselemente (23) bzw. der Implantatplatten (11) jeweils mit einem insbesondere zangenartigen Distraktionsinstrument koppelbar sind.

2. Instrumentensystem nach Anspruch 1,
**dadurch gekennzeichnet ,**
**dass** die Probeeinheit (15), die Bearbeitungseinheit (19) und die Halteeinheit (25) jeweils an ihrer während der Operation dem Operationsfeld abgewandten Rückseite mit Kopplungsabschnitten (61) für das Distraktionsinstrument versehen sind.

3. Instrumentensystem nach Anspruch 1 oder 2,
**dadurch gekennzeichnet ,**
**dass** die Probeeinheit (15) eine einstellbare Tiefenlehre (27) zur Festlegung einer Referenzposition für das Implantat in anterior-posterior-Richtung aufweist.

4. Instrumentensystem nach Anspruch 3,
**dadurch gekennzeichnet ,**
**dass** die Bearbeitungseinheit (19) und/oder die Halteeinheit (25) ebenfalls eine einstellbare Tiefenlehre (27) aufweisen, die entsprechend der Referenzeinstellung der Probeeinheit (15) einstellbar ist.

5. Instrumentensystem nach Anspruch 3 oder 4,
**dadurch gekennzeichnet ,**
**dass** alle Tiefenlehren (27) zumindest im Wesentlichen baugleich ausgeführt sind.

6. Instrumentensystem nach einem der Ansprüche 3 bis 5,
**dadurch gekennzeichnet ,**
**dass** die Tiefenlehre (27) einen kaudalen und einen kranialen Abschnitt umfasst, die unabhängig voneinander einstellbar sind.

7. Instrumentensystem nach einem der Ansprüche 3 bis 6
**dadurch gekennzeichnet ,**
**dass** die Tiefenlehre (27) zumindest bereichsweise nach Art einer in anterior-posterior-Richtung relativ zur Probeeinheit (15) verstellbaren Einstellschraube ausgebildet ist.

8. Instrumentensystem nach einem der Ansprüche 3 bis 7,
**dadurch gekennzeichnet ,**
**dass** die Tiefenlehre (27) über eine Stift/Nut-Führung mit der Probeeinheit (15) gekoppelt ist.

9. Instrumentensystem nach einem der Ansprüche 3 bis 8,
**dadurch gekennzeichnet ,**
**dass** die Tiefenlehre (27) einen insbesondere mittels eines Schraubendrehers um eine im Wesentlichen parallel zur anterior-posterior-Richtung verlaufende Achse drehbaren Verstellabschnitt (29) aufweist, an dem eine zumindest bereichsweise wendel- oder schraubenförmig um die Drehachse herum verlaufende Verstellnut (31) für einen an der Probeeinheit (15) angeordneten Führungsstift (33) ausgebildet ist.

10. Instrumentensystem nach Anspruch 9,
**dadurch gekennzeichnet ,**
**dass** die Verstellnut (31) mehrere voneinander beabstandete, vorgegebenen Tiefenpositionen entsprechende Rastvertiefungen (35) für den Führungsstift (33) aufweist.

11. Instrumentensystem nach einem der Ansprüche 3 bis 10,
**dadurch gekennzeichnet ,**
**dass** die Tiefenlehre (27) eine insbesondere im Wesentlichen senkrecht zur anterior-posterior-Richtung verlaufende, während der Operation die Position der Probeeinheit (15) relativ zum Wirbelkörper festlegende Anschlagplatte (37) aufweist.

12. Instrumentensystem nach Anspruch 11,
**dadurch gekennzeichnet ,**
**dass** ein in anterior-posterior-Richtung fest mit der Anschlagplatte (37) verbundener Verstellabschnitt (29) der Tiefenlehre (27) relativ zur Anschlagplatte (37) verdrehbar ist.

13. Instrumentensystem nach einem der vorhergehenden Ansprüche,
**dadurch gekennzeichnet ,**
**dass** die Bearbeitungseinheit (19) zusätzlich zu den Arbeitselementen (23) wenigstens eine Führungshülse (39) für ein Bearbeitungsinstrument, insbesondere einen Fräser oder Bohrer, umfasst, die zur Festlegung einer Bearbeitungsposition mit der Bearbeitungseinheit (19) koppelbar ist, vorzugsweise derart, dass die Führungshülse (39) in Richtungen senkrecht zu einer in oder geneigt zur anterior-posterior-Richtung verlaufenden Bearbeitungsachse bezüglich der Bearbeitungseinheit (19) fest gehalten ist, wobei bevorzugt ein Bearbeitungswinkel zwischen der Bearbeitungsachse und der anterior-posterior-Richtung einem Neigungswinkel der Implantatplatten (11) entspricht, der vorzugsweise im Bereich von 2° bis 7° liegt.

14. Instrumentensystem nach Anspruch 13,
**dadurch gekennzeichnet ,**
**dass** die Führungshülse (39) sowohl zur kaudalen als auch zur kranialen Bearbeitung ausgebildet und hierzu zunächst mit dem einen und anschließend mit dem anderen Arbeitselement (23) koppelbar ist.

15. Instrumentensystem nach Anspruch 13 oder 14,
**dadurch gekennzeichnet ,**
**dass** die Bearbeitungseinheit (19) bereichsweise einen tunnelartigen Aufnahmeraum begrenzt, in den die Führungshülse (39) zumindest teilweise in anterior-posterior-Richtung einschiebbar ist.

16. Instrumentensystem nach einem der vorhergehenden Ansprüche,
**dadurch gekennzeichnet ,**
**dass** die Bearbeitungseinheit (19) ein separates Verriegelungselement (41) umfasst, mit dem ein mittels des Distraktionsinstrumentes hergestellter Abstand zwischen den Arbeitselementen (23) fixierbar ist.

17. Instrumentensystem nach Anspruch 16,
**dadurch gekennzeichnet ,**
**dass** das Verriegelungselement (41) wenigstens einen in anterior-posterior-Richtung zwischen die Arbeitselemente (23) schiebbaren Verriegelungsabschnitt (43) mit einem gestuften oder treppenartig verlaufenden Höhenprofil (45) aufweist.

18. Instrumentensystem nach Anspruch 16 oder 17,
**dadurch gekennzeichnet ,**
**dass** der Verriegelungsabschnitt (43) gabelförmig ausgebildet ist und zwei identische Höhenprofile (45) aufweisende Verriegelungsarme (43) umfasst, die jeweils zwischen einander zugewandte Längskantenabschnitte (47) der vorzugsweise in diesem Bereich halbschalen- oder trogartig ausgebildeten Arbeitselemente (23) schiebbar sind.
